# EUROPÄISCHE PATENTSCHRIFT

(11) **EP 3 272 423 B1**
(45) Veröffentlichungstag und Bekanntmachung des Hinweises auf die Patenterteilung: **19.12.2018**
(21) Anmeldenummer: 16180422.4
(22) Anmeldetag: 20.07.2016
(51) Int. Cl.: B05B 1/30, B05B 1/34, B05B 1/12, B05B 11/00

(54) **AUSTRAGKOPF UND SPENDER MIT EINEM AUSTRAGKOPF**
DISCHARGE HEAD AND DISPENSER WITH A DISCHARGE HEAD
TETE DISTRIBUTRICE ET DISTRIBUTEUR COMPRENANT UNE TETE DISTRIBUTRICE

(43) Veröffentlichungstag der Anmeldung: 24.01.2018
(73) Patentinhaber: Aptar Radolfzell GmbH, 78315 Radolfzell (DE)
(72) Erfinder: Baumann, Tobias, 78465 Konstanz (DE)
(74) Vertreter: Patentanwaltskanzlei Cartagena

(56) Entgegenhaltungen:
- EP-A2- 0 729 792
- WO-A1-2014/138421
- US-A- 4 911 361
- US-A1- 2016 023 221

## Beschreibung

### ANWENDUNGSGEBIET UND STAND DER TECHNIK

Die Erfindung betrifft einen Flüssigkeitsspender nach dem Oberbegriff von Anspruch 1, der für den wahlweisen Austrag von Flüssigkeit einerseits in Form eines zerstäubten Sprühstrahls und andererseits in Form eines unzerstäubten Flüssigkeitsstroms oder in Form von Einzeltropfen ausgebildet ist.

Aus der WO 2015/106776 A1 ist ein Spender zum Austrag von Flüssigkeiten mit zwei Auslasskanälen bekannt, die jeweils über eine eigene Auslassöffnung verfügen. Durch eine Drehbewegung kann gesteuert werden, in welchen der Auslasskanäle die auszutragende Flüssigkeit strömt.

Aus den US 2016/0023221 A1 ist eine als Quetschflasche ausgebildete Getränkeflasche bekannt, die über einen Auslass verfügt, der zwischen zwei Konfigurationen umstellbar ist, um einen Flüssigkeitsstrom und einen Flüssigkeitsnebel austragen zu können.

### AUFGABE UND LÖSUNG

Aufgabe der Erfindung ist es, einen Flüssigkeitsspender zur Verfügung zu stellen, der auf besonders bequeme Art und Weise den Austrag von Flüssigkeit mit unterschiedlicher Austragcharakteristik gestattet.

Der erfindungsgemäße Flüssigkeitsspender weist eine Basiseinheit mit einem Flüssigkeitsspeicher zur Aufnahme von Flüssigkeit vor dem Austrag und einen Austragkopf auf.

Der Flüssigkeitsspender kann über eine Pumpeinrichtung verfügen, die durch eine translative Relativbewegung des Austragkopfes gegenüber der Basiseinheit betätigt werden kann und Flüssigkeit aus dem Flüssigkeitsspeicher in den Austragkopf fördert. Bei einer Gestaltung mit Pumpeinrichtung bewirkt somit ein Niederdrücken des Austragkopfes gegenüber der Basiseinheit den Austrag. Die Pumpe ist vorzugsweise an der Basiseinheit vorgesehen und weist ein Auslassrohr auf, auf das der Austragkopf aufgesetzt ist. Gemeinsam mit dem Auslassrohr wird beim Niederdrücken des Austragkopfes ein Pumpenkolben verlagert, der Flüssigkeit durch das Auslassrohr in den Austragkopf fördert.

Der Flüssigkeitsspeicher kann stattdessen auch als Druckspeicher ausgebildet sein und der Flüssigkeitsspender über ein Auslassventil verfügen, das durch eine translative Relativbewegung des Austragkopfes gegenüber der Basiseinheit betätigt werden kann und Flüssigkeit aus dem Flüssigkeitsspeicher in den Austragkopf fördert. Die Gestaltung mit einem Flüssigkeitsspeicher, in welchem die Flüssigkeit unter Druck gelagert ist, wird als vorteilhaft angesehen, da sie einen langen ununterbrochenen Austrag gestattet. Die Verlagerung des Austragkopfes gegenüber der Basiseinheit bewirkt hier ein Öffnen des Auslassventils, so dass die druckbeaufschlagte Flüssigkeit aus dem Flüssigkeitsspeicher in den Austragkopf strömen kann.

Der Austragkopf ist zur Befestigung auf der Basiseinheit des Flüssigkeitsspenders vorgesehen, welche den Flüssigkeitsspeicher umfasst. Er weist ein Gehäuse auf, das zur ortsfesten oder linearbeweglichen oder linear- und drehbeweglichen Befestigung an der Basiseinheit des Flüssigkeitsspenders ausgebildet ist. Er weist einen Flüssigkeitsauslass auf, durch den Flüssigkeit aus dem Flüssigkeitsspeicher in eine umgebende Atmosphäre abgegeben werden kann, sowie einen Flüssigkeitseinlass, durch den hindurch Flüssigkeit aus dem Flüssigkeitsspeicher zum Flüssigkeitsauslass geleitet werden kann.

Der Flüssigkeitsauslass verfügt über einen die Außenfläche des Gehäuses durchdringenden Auslasskanal. Innerhalb dieses Auslasskanals ist ein Innenteil angeordnet, an deren in Richtung der umgebenden Atmosphäre weisende Stirnseite eine Sprühöffnung als Ende eines Sprühkanals vorgesehen ist. Das Innenteil und der Auslasskanal sind gegeneinander verlagerbar, so dass in einer ersten Relativstellung ein Umgehungskanal zwischen einer Innenwandung des Auslasskanals und einer Außenfläche des Innenteils entsteht, deren Strömungswiderstand geringer als der des Sprühkanals ist, und in einer zweiten Relativstellung dieser Umgehungskanal zumindest teilweise geschlossen ist, so dass der Strömungswiderstand des Umgehungskanals größer als der des Sprühkanals ist.

Bei einem erfindungsgemäßen Austragkopf sind somit zwei unterschiedliche Flüssigkeitspfade vorgesehen, die beide in einen gemeinsamen Flüssigkeitsauslass des Austragkopfes münden. Der eine dieser Flüssigkeitspfade führt durch den Sprühkanal, welcher das im Auslasskanal angeordnete Innenteil durchdringt. Dieser Sprühkanal ist durch eine geeignete geometrische Gestaltung zum Erzeugen eines Sprühstrahls ausgebildet, beispielsweise durch das Vorsehen einer Wirbelkammer stromaufwärts der Sprühöffnung oder durch eine oder mehrere entsprechend fein dimensionierte Sprühdüsen. Der zweite Flüssigkeitspfad führt durch den Auslasskanal, jedoch nicht durch das Innenteil im Auslasskanal. Dieser zweite Sprühpfad, der am Innenteil vorbeiströmt, dient zum Austrag der Flüssigkeit mit geringerer kinetischer Energie, so dass sich ein kontinuierlicher Flüssigkeitsfluss oder Tropfenbildung einstellt.

Das im Auslasskanal angeordnete Innenteil, welches vom Sprühkanal durchdrungen wird, ist relativ zum Auslasskanal beweglich, so dass das Verhältnis zwischen dem Strömungswiderstand des ersten Flüssigkeitspfades durch den Sprühkanal hindurch zum Strömungswiderstand des zweiten Flüssigkeitspfades durch den Umgehungskanal hindurch beeinflussen lässt. In Abhängigkeit der Relativlage ist der Strömungswiderstand auf dem ersten oder dem zweiten Flüssigkeitspfad geringer, so dass zumindest der überwiegende Teil der Flüssigkeit entlang dieses Pfades mit geringerem Strömungswiderstand ausgetragen wird. Strömt die Flüssigkeit durch den Sprühkanal, so wird sie in Form eines Sprühstrahls am Flüssigkeitsauslass ausgetragen. Strömt die Flüssigkeit durch den Umgehungskanal, so verlässt sie den Flüssigkeitsauslass in Form eines kontinuierlichen Flüssigkeitsstroms geringer kinetischer Energie oder in Form von Einzeltropfen.

Durch die Gestaltung des Austragkopfes mit einem einheitlichen Flüssigkeitsauslass ist eine sehr intuitive und einfache Benutzung möglich. Dies wird durch die im Weiteren noch erläuterten Gestaltungsvariationen zur Betätigung noch verstärkt.

Bei einer Weiterbildung ist vorgesehen, dass in der ersten Relativstellung der Umgehungskanal geschlossen ist, so dass Flüssigkeit nur durch den Sprühkanal in die umgebende Atmosphäre gelangen kann, und/oder dass in der zweiten Relativstellung ein Einlass in den Sprühkanal geschlossen ist, so dass Flüssigkeit nur durch den Umgehungskanal in die umgebende Atmosphäre gelangen kann.

Obwohl es grundsätzlich nicht zwingend erforderlich ist, dass der Umgehungskanal bzw. der Strömungskanal in den jeweiligen Endlagen komplett verschlossen wird, ist dies von Vorteil, um jeweils die Gesamtheit der Flüssigkeit durch den nicht verschlossenen Kanal zu leiten.

Bei einer Variante wird die Innenwandung des Auslasskanals unmittelbar durch Oberflächen des Gehäuses gebildet und das Innenteil ist gegenüber dem Gehäuse verlagerbar.

Als Gehäuse im Sinne der Erfindung werden alle Teile des Austragkopfes verstanden, die zueinander ortsfest die Haupteinheit des Austragkopfes bilden. Die genannte Variante der Erfindung sieht vor, dass die Innenwandung des Auslasskanals durch Oberflächen des Gehäuses gebildet wird. Das Innenteil ist dagegen bei dieser Gestaltung nicht Teil des Gehäuses, sondern gegenüber dem Gehäuse verlagerbar, um in Abhängigkeit der Relativstellung vom Gehäuse den Flüssigkeitspfad zu beeinflussen.

Bei einer anderen Variante wird die Innenwandung des Auslasskanals durch eine gegenüber dem Gehäuse verlagerbare Auslasshülse gebildet und ist damit auch gegenüber dem Innenteil verlagerbar, welches Teil des Gehäuses ist.

Bei dieser alternativen Variante ist somit das Innenteil Teil des Gehäuses und gegenüber den anderen Gehäuseteilen unbeweglich, während die Innenwandung des Auslasskanals verlagerbar ist, insbesondere indem sie Teil einer Auslasshülse ist, die gegenüber dem Gehäuse verschoben werden kann.

Zur Erzeugung des gewünschten Sprühbildes im Sprühkanal weist dieser vorzugsweise eine Wirbelkammer auf. In eine solche Wirbelkammer strömt die Flüssigkeit in einer exzentrischen Richtung, so dass sie einen Drall in die Flüssigkeit innerhalb der Wirbelkammer einbringt. Dieser Drall bleibt erhalten und führt beim Austrag der Flüssigkeit zur Ausbildung eines Sprühkegels.

Zur baulichen Realisierung einer Geometrie zur Erzeugung eines Sprühstrahls ist es vorteilhaft, wenn das Innenteil einen Außenwandungsabschnitt bildet, in den ein Sprühbauteil eingesetzt ist. Dieses Sprühbauteil kann insbesondere zumindest abschnittsweise eine Innenwandung der Wirbelkammer bilden, in der der Sprühstrahl erzeugt wird.

Der Umgehungskanal, durch den die Flüssigkeit zur Ausbildung eines stetigen Flüssigkeitsstroms oder zur Tropfenbildung strömt, verläuft zwischen der Innenwandung des Auslasskanals und einer Außenwandung des Innenteils. Vorzugsweise ist dieser Umgehungskanal als ringförmiger Kanal ausgebildet, der das Innenteil umgibt.

Die Relativbeweglichkeit des Innenteils und des Auslasskanals ist vorzugsweise eine translative, also nicht-rotative Beweglichkeit. Hierunter wird verstanden, dass keine eine Drehachse definierenden Bauteile vorgesehen sind, sondern stattdessen das Innenteil oder der Auslasskanal entlang einer Spur schiebebeweglich sind, vorzugsweise linearbeweglich. Insbesondere von Vorteil ist es, wenn die Relativbeweglichkeit fluchtend mit der Haupterstreckungsrichtung des Auslasskanals und/oder der Austragrichtung der Flüssigkeit erfolgt.

Die Relativverlagerung des Innenteils gegenüber dem Auslasskanal kann auf verschiedene Weisen hergestellt werden. Eine mögliche Gestaltung sieht vor, dass das Innenteil und der Auslasskanal über einen Gewindetrieb translativ beweglich sind, so dass die vorzugsweise lineare Relativverlagerung durch eine rotative Stellbewegung am Auslasskanal oder am Innenteil erfolgt.

Es kann zum Zwecke der Relativverlagerung insbesondere eine Schaltfläche zur manuellen Betätigung vorgesehen sein, mittels derer die Relativlage des Auslasskanals gegenüber dem Innenteil veränderbar ist.

Die Schaltfläche kann unmittelbar an einer gegenüber dem Gehäuse beweglichen Auslasshülse oder am gegenüber dem Gehäuse beweglichen Innenteil vorgesehen sein. Bei einer solchen Gestaltung ist die Schaltfläche somit ortsfest zur Auslasshülse oder zum Innenteil, so dass eine mit einer Kraftbeaufschlagung der Schaltfläche einhergehende Verlagerung der Auslasshülse oder des Innenteils gegenüber dem Gehäuse unmittelbar auch die Relativstellung des Innenteils zum Auslasskanal beeinflusst.

Alternativ kann die Schaltfläche mittels eines Getriebes mit dem gegenüber dem Gehäuse beweglichen Auslasskanal oder mit dem gegenüber dem Gehäuse beweglichen Innenteil gekoppelt sein. Bei dieser alternativen Gestaltung ist ein Getriebe vorgesehen, durch welches es beispielsweise möglich ist, eine Richtungsumlenkung zu bewirken. So kann beispielsweise die Bewegung der Schaltfläche in einer Richtung zu einer Relativverlagerung des Innenteils und der Auslasshülse zueinander in einer hiervon abweichenden zweiten Richtung erfolgen.

Es kann eine Rückstellfeder vorgesehen, die zwischen dem Innenteil und dem Auslasskanal wirkt, so dass das Innenteil und der Auslasskanal stets in Richtung der ersten oder der zweiten Relativstellung kraftbeaufschlagt sind und durch Kraftbeaufschlagung der Schaltfläche gegen die Kraft der Rückstellfeder verlagert werden.

Die Rückstellfeder führt dazu, dass beim Wegfall einer Kraftbeaufschlagung der Schaltfläche der Austragkopf wieder seine Ausgangsstellung einnimmt. Dies kann zum einen derart realisiert sein, dass die Relativanordnung des Innenteils und des Auslasskanals in jener Stellung, die durch Kraftbeaufschlagung der Schaltfläche bewirkt wird, arretierbar ist. Vorteilhaft ist jedoch eine Gestaltung, bei der die Kraftbeaufschlagung der Schaltfläche nicht nur die Verlagerung des Innenteils und des Auslasskanals gegen die Kraft der Feder bewirkt, sondern auch den Austrag selbst bewirkt. Somit kann ein Niederdrücken der Schaltfläche sowohl zunächst die Verlagerung des Innenteils und des Auslasskanals gegeneinander bewirken und nachfolgend den Austragvorgang entlang des Flüssigkeitspfades, der sich durch die Kraftbeaufschlagung der Schaltfläche ergibt.

Der Austragkopf kann über ein variables Innenvolumen für Flüssigkeit verfügen, welches durch Einschieben der Auslasshülse vergrößert wird. Durch ein variables Innenvolumen wird erreicht, dass insbesondere bei einer Gestaltung mit gegenüber dem Gehäuse verlagerbarer Auslasshülse die Verlagerung dieser Auslasshülse in das Gehäuse hinein nicht zu einem unerwünschten Flüssigkeitsaustrag führt. Durch das variable Volumen kann die anderenfalls aus der Auslasshülse verdrängte Flüssigkeit im Austragkopf aufgenommen werden.

Der Auslasskanal kann eine sich in Richtung der umgebenden Atmosphäre verjüngende Formgebung aufweisen. Der Auslasskanal mit der sich verjüngenden Formgebung ist besonders geeignet, um den verschließbaren Umgehungskanal zu realisieren. Das Innenteil weist hierbei einen Außendurchmesser auf, der geringer als der maximale lichte Querschnitt des Auslasskanals ist, jedoch größer als der minimale lichte Querschnitt. Somit kann eine Verlagerung des Innenteils gegen den sich verjüngenden Abschnitt zum Schließen des Umgehungskanals genutzt werden.

Die Schaltfläche zur Verlagerung des Innenteils gegenüber dem Auslasskanal ist vorzugsweise auf einer der Basiseinheit abgewandten Seite des Austragkopfes vorgesehen. Die Schaltfläche ist weiterhin vorzugsweise gegenüber dem Gehäuse des Austragkopfes in einer Richtung beweglich, die der translativen Relativbewegungsrichtung des Austragkopfes gegenüber der Basiseinheit entspricht (+/-20°).

Obwohl grundsätzlich auch andere Gestaltungen möglich sind, werden Flüssigkeitsspender als vorteilhaft angesehen, bei denen ein Niederdrücken des Austragkopfes den Austrag bewirkt. An der Oberseite des Austragkopfes ist zu diesem Zweck eine Betätigungsfläche vorgesehen. Wenn die Schaltfläche ebenfalls dort vorgesehen ist, so gestattet es dies, die Schaltfläche zur Beeinflussung der Austragcharakteristik mit dem gleichen Finger zu bedienen, der auch den Austragkopf niederdrückt. Ein besonderer Vorteil ergibt sich, wenn die Relativbeweglichkeit der Schaltfläche gegenüber dem Gehäuse des Austragkopfes im Wesentlichen mit der Relativbeweglichkeit des Austragkopfes übereinstimmt, da hierdurch erzielt werden kann, dass eine einheitliche Bewegung zunächst den Strömungspfad im Austragkopf beeinflusst und anschließend den Austragvorgang startet. Bei einer solchen Gestaltung kann vorgesehen sein, dass die Oberseite des Austragkopfes zweigeteilt ist. Die Kraftbeaufschlagung in einem ersten Teil, in dem die Schaltfläche vorgesehen ist, führt zur beschriebenen Änderung des Strömungspfades und anschließend zum Austrag. Die Kraftbeaufschlagung des Austragkopfes neben der Schaltfläche führt zu einem Austragvorgang, ohne dass zuvor der Strömungspfad verstellt worden ist.

Bei einer besonderen Gestaltung ist der Austragkopf gegenüber der Basiseinheit um eine Drehachse drehbar ausgebildet. Dabei ist ein Getriebe vorgesehen, mittels die Drehbewegung des Austragkopfs eine Relativverlagerung des Auslasskanals gegenüber dem Innenteil bewirkt. Das Getriebe umfasst ein Führungselement mit winkelabhängiger Beabstandung von der Drehachse, welches am Innenteil oder der Auslasshülse des Austragkopfes oder an der Basiseinheit vorgesehen ist, und einen Führungsgleiter, der im Eingriff mit dem Führungselement ist und der gegenüberliegend zum Führungselement an der Basiseinheit bzw. am Innenteil oder an der Auslasshülse des Austragkopfes vorgesehen ist.

Die winkelabhängige Beabstandung ist vorzugsweise in Art einer Spiralabschnittsform vorgesehen. Wenn der Führungsgleiter entlang des Führungselements gleitet, ändert er dadurch auch seinen Abstand zur Drehachse, also eine radiale Verlagerung. Alternativ könnte bei radial feststehendem Führungsgleiter auch das Führungselement eine radiale Verlagerung erfahren. Diese radiale Verlagerung kann genutzt werden, um die Auslasshülse oder das Innenteil radial zu verlagern.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt ein erstes Ausführungsbeispiel eines Flüssigkeitsspenders mit Austragkopf in einer geschnittenen Darstellung.
Fig. 2 zeigt den Austragkopf des Flüssigkeitsspenders in einer perspektivischen Darstellung, aus der die Oberseite des Austragkopfes ersichtlich ist.
Fig. 3 und 4 zeigen den Austragkopf in geschnittener Darstellung in zwei unterschiedlichen Konfigurationen.
Fig. 5 zeigt ein zweites Ausführungsbeispiel eines Flüssigkeitsspenders mit Austragkopf in einer teilgeschnittenen Darstellung.
Fig. 6 und 7 zeigen den Austragkopf in geschnittener Darstellung in zwei unterschiedlichen Konfigurationen.
Fig. 8 und 9 zeigen ein zweites Ausführungsbeispiel eines Flüssigkeitsspenders mit Austragkopf in einer teilgeschnittenen Darstellung in zwei unterschiedlichen Konfigurationen.

### DETAILLIERTE BESCHREIBUNG DER AUSFÜHRUNGSBEISPIELE

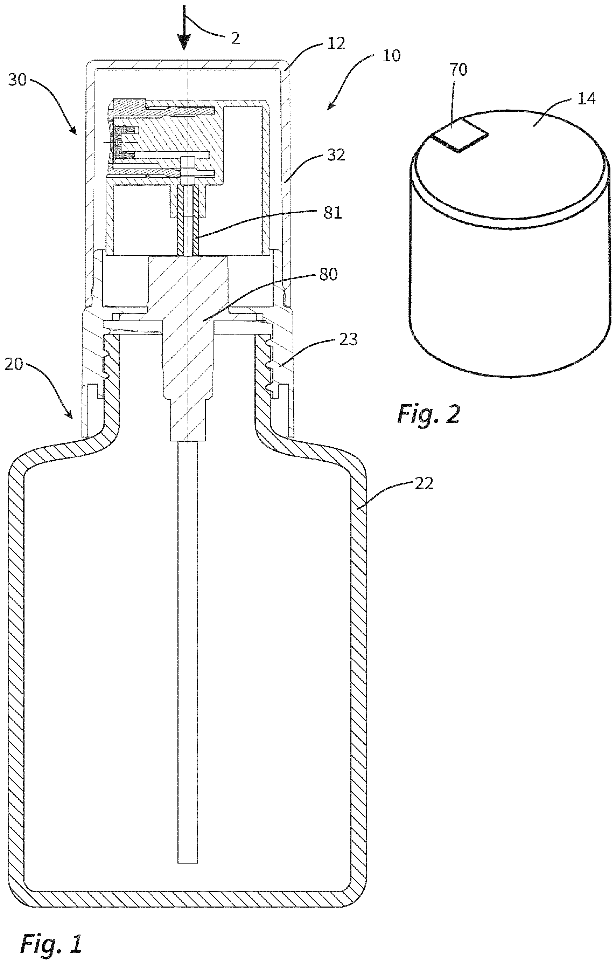

Fig. 1 zeigt in Gesamtdarstellung ein erstes Ausführungsbeispiel eines erfindungsgemäßen Flüssigkeitsspenders 10. Dieser Flüssigkeitsspender 10 verfügt über eine Basiseinheit 20, umfassend einen Flüssigkeitsspeicher 22 sowie ein darauf aufgesetztes Gehäuseteil 23, die gemeinsam eine Pumpeinrichtung 80 festlegen, an deren oberen Ende ein Auslassrohr 81 vorgesehen ist. Auf dieses aufgesetzt ist ein Austragkopf 30, der im Zustand der Fig. 1 von einer Schutzkappe 12 überdeckt wird. Dieser Austragkopf 30 kann in Richtung des Pfeils 2 zum Zwecke eines Flüssigkeitsaustrages niedergedrückt werden.

Der Austragkopf 30 ist dafür vorgesehen, Flüssigkeit wahlweise in Form eines Sprühstrahls oder in Form eines stetigen Flüssigkeitsstroms bzw. Tropfen auszutragen.

Um benutzerseitig dies beeinflussen zu können, ist der Austragkopf in der in Fig. 2 dargestellten Weise mit einer Schaltfläche 70 im Bereich einer Betätigungsfläche 14 versehen. Die Relativanordnung dieser Schaltfläche 70 relativ zum Gehäuse 32 des Austragkopfes 30 und der Betätigungsfläche 14 bestimmt, in welcher Form die Flüssigkeit ausgetragen wird.

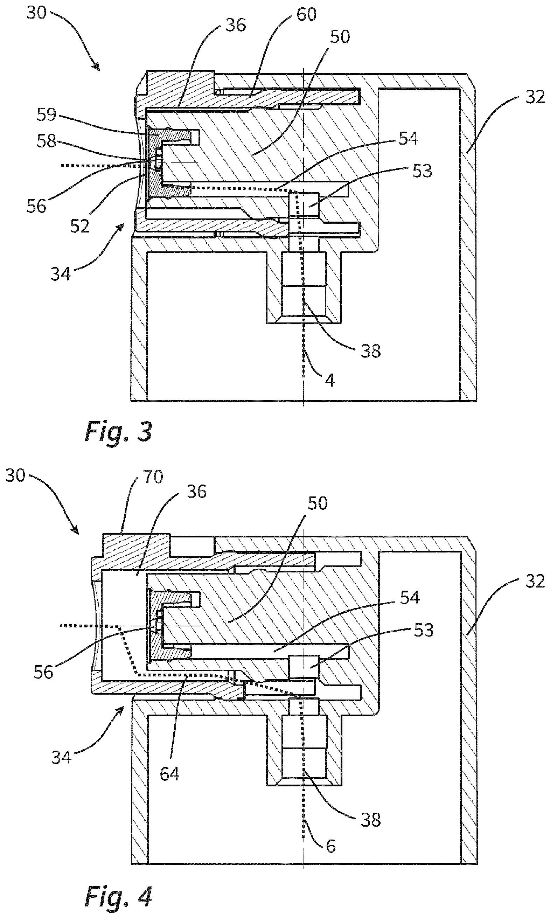

Bezug nehmend auf die Fig. 3 und 4 werden die unterschiedlichen Konfigurationen des Austragkopfes 30 erläutert.

Der Austragkopf 30 verfügt über einen Flüssigkeitseinlass 38, der in der bereits beschriebenen Weise am Auslassrohr 81 der Pumpeinrichtung 80 befestigt wird. Vom Flüssigkeitseinlass 38 wird die Flüssigkeit zu einem einheitlichen Flüssigkeitsauslass 34 geleitet, den die auszutragende Flüssigkeit beim Austrag durchquert. In der Sprühkonfiguration der Fig. 3 strömt die Flüssigkeit zu diesem Zweck entlang eines Flüssigkeitspfades 4, der durch einen radialen Einlass 53 in ein Innenteil 50 des Austragkopfes 30 führt, welches ortsfest zum Gehäuse 32 des Austragkopfes angeordnet ist. Das Innenteil wird von einem Sprühkanal 54 durchdrungen, entlang dessen der Flüssigkeitspfad 4 sich bis zu einer Wirbelkammer 58 erstreckt. In dieser wird die auszutragende Flüssigkeit mit einem Drall versehen, so dass sie durch eine Sprühöffnung 56 an der Stirnseite 52 des Innenteils 50 in Form eines Sprühstrahls austritt, ohne nach dem Austritt mit anderen Gehäuseteilen nochmals in Kontakt zu gelangen. Der Weg der Flüssigkeit in der Sprühkonfiguration ist durch den Flüssigkeitspfad 4 in der Fig. 3 verdeutlicht.

Das Innenteil 50 ist innerhalb eines Auslasskanals 36 angeordnet, der gegenüber dem Gehäuse 32 des Austragkopfes 30 translativ linear in horizontaler Richtung verlagerbar ist.

Fig. 4 zeigt die Tropferkonfiguration des Austragkopfes 30. In dieser Tropferkonfiguration ist eine Auslasshülse 60, deren Innenseite den Auslasskanal 36 bildet, derart verschoben, dass ein Umgehungskanal 64 geöffnet wird. Der Strömungspfad der Flüssigkeit ist nunmehr der Flüssigkeitspfad 6, der am Innenteil 50 vorbei innerhalb des Auslasskanals 36 bis zum Flüssigkeitsauslass 34 führt.

Da der lichte Querschnitt des Umgehungskanals 64 deutlich größer als der der Sprühöffnung 56 ist, strömt die Flüssigkeit mit geringerer Geschwindigkeit zum Flüssigkeitsauslass 34 und kann somit hier vergleichsweise drucklos abgegeben werden.

Bei dem Ausführungsbeispiel der Fig. 1 bis 4 wird der Umgehungskanal 64 in der Sprühkonfiguration der Fig. 3 geschlossen. In der Tropferkonfiguration der Fig. 4 ist der Sprühkanal 54 jedoch nach wie vor offen. Aufgrund des erhöhten Strömungswiderstandes des Sprühkanals 54 und der Sprühöffnung 56 strömt dennoch die gesamte Flüssigkeit oder nahezu die gesamte Flüssigkeit in der Tropferkonfiguration der Fig. 4 durch den Umgehungskanal 64.

Bei alternativen Gestaltungen kann jedoch auch vorgesehen sein, dass der Sprühkanal 54 in der Tropferkonfiguration komplett geschlossen wird. Dies ist baulich einfach durch eine entsprechende Verlängerung der Auslasshülse 60 und die Einbringung einer radialen Durchbrechung zu erzielen, so dass die verlängerte Auslasshülse in der Konfiguration der Fig. 4 mittels dieser Verlängerung den Zugang zum Einlass 53 des Innenteils 50 verschließt.

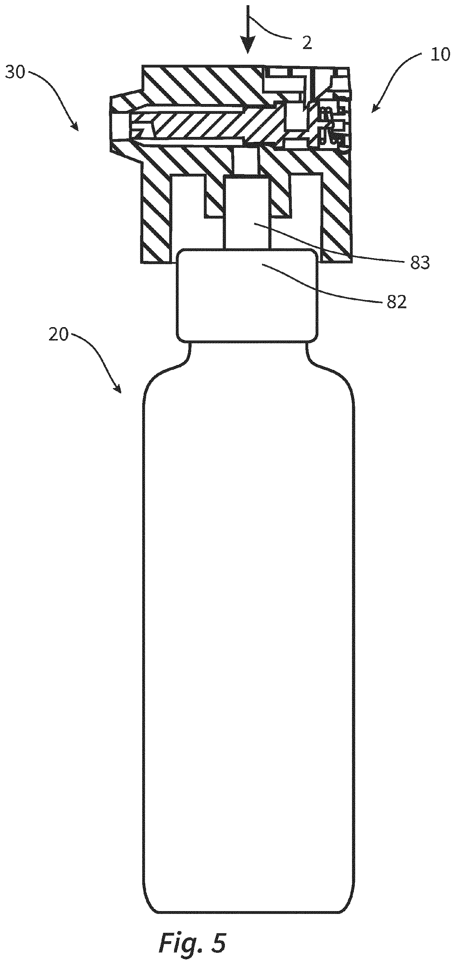

Fig. 5 zeigt eine alternative Gestaltung. Bei dem hier dargestellten Flüssigkeitsspender 10 sind wiederum eine Basiseinheit 20 und ein in Richtung des Pfeils 2 verlagerbarerAustragkopf 30 vorgesehen. Bei dieser Gestaltung ist die Basiseinheit 20 jedoch mit einem als Druckspeicher versehenen Flüssigkeitsspeicher 22 versehen. Statt der Pumpeinrichtung 80 kommt daher eine nicht näher dargestellte Ventileinrichtung 82 zum Einsatz, die durch Verlagerung eines Auslassstutzens 83 in Richtung des Pfeils 2 geöffnet werden kann.

Grundsätzlich kann jedoch auch bei dieser Gestaltung eine Basiseinheit mit Pumpeinrichtung entsprechend dem vorangestellten Ausführungsbeispiel Verwendung finden. Ebenso kann auch beim vorangestellten Ausführungsbeispiel statt des Flüssigkeitsspeichers mit Pumpeinrichtung ein Druckspeicher mit Ventileinrichtung Verwendung finden.

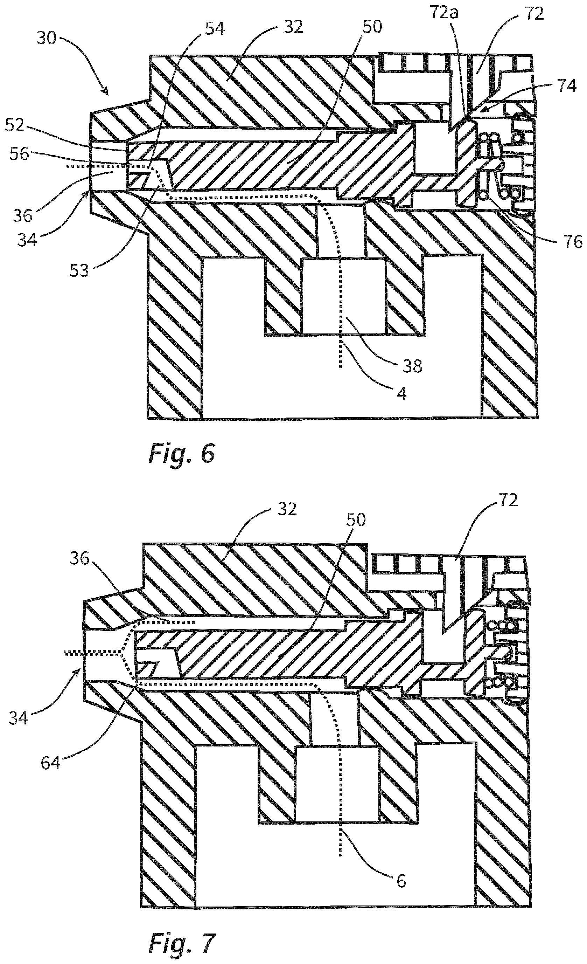

Bezug nehmend auf die Fig. 6 und 7 werden die beiden Konfigurationen des Austragkopfes 30 erläutert.

In der Konfiguration der Fig. 6 ist der Austragkopf zur Erzeugung eines Sprühstrahls konfiguriert. Die durch den Flüssigkeitseinlass 38 strömende Flüssigkeit gelangt in den Einlass 53 des Sprühkanals 54, da kein anderer Pfad zum Flüssigkeitsauslass 34 führt. Die Flüssigkeit wird durch die vergleichsweise enge Sprühöffnung 56 am Ende des Sprühkanals 54 ausgetragen. Wenngleich eine hierfür besonders angepasste geometrische Gestaltung in den Figuren nicht dargestellt wird, könnte hier auch eine Wirbelkammer entsprechend dem ersten Ausführungsbeispiel Verwendung finden. Ebenfalls möglich ist eine Gestaltung mit einer oder mehreren feinen Düsenöffnungen zur Erzeugung eines Sprühstrahls.

In der Konfiguration der Fig. 7 ist das Innenteil 50 gegenüber dem Gehäuse 32 bezogen auf die Darstellungen nach rechts verlagert. Hierdurch wird der Umgehungskanal 64 geöffnet, so dass nunmehr die Flüssigkeit wiederum vergleichsweise drucklos zum Flüssigkeitsauslass 34 strömen kann. Somit ist in Hinblick auf das Innenteil 50 und den Auslasskanal 36 die Gestaltung gegenüber dem ersten Ausführungsbeispiel umgekehrt. Bei dieser Gestaltung der Fig. 5 bis 7 ist das Innenteil 50 gegenüber dem Gehäuse 32 linear verlagerbar, während der Auslasskanal 36 gegenüber dem Gehäuse 32 ortsfest verbleibt.

Die Besonderheit dieses Ausführungsbeispiels liegt jedoch insbesondere in der Art der Betätigung. An der Oberseite des Austragkopfes 30 ist eine Schaltfläche 72 vorgesehen, die ebenfalls in Richtung des Pfeils 2 niedergedrückt werden kann. Diese ist über eine schiefe Ebene 72a mit einem rückwärtigen Ende des Innenteils 50 gekoppelt. Zudem ist das Innenteil 50 mittels einer Rückstellfeder 76 in Richtung der Sprühkonfiguration der Fig. 6 kraftbeaufschlagt.

Wird nun in der in Fig. 7 verdeutlichten Weise die Schaltfläche 72 gegenüber dem Gehäuse 32 niedergedrückt, so wird unter gleichzeitiger Komprimierung der als Druckfeder ausgestalteten Rückstellfeder 76 das Innenteil 50 nach rechts verlagert und somit der Umgehungskanal 64 geöffnet.

Damit ist es möglich, in Abhängigkeit des konkreten Ortes der Kraftbeaufschlagung des Austragkopfes eine von beiden Konfigurationen zu wählen. Wird der Austragkopf im Bereich der Schaltfläche 72 niedergedrückt, so führt dies zu der Ausgabe eines weitgehend drucklosen Flüssigkeitsstroms. Erfolgt die Betätigung abseits der Schaltfläche 72, so ist die Sprühkonfiguration der Fig. 6 gegeben, so dass die Flüssigkeit in Form eines Sprühstrahls ausgetragen wird.

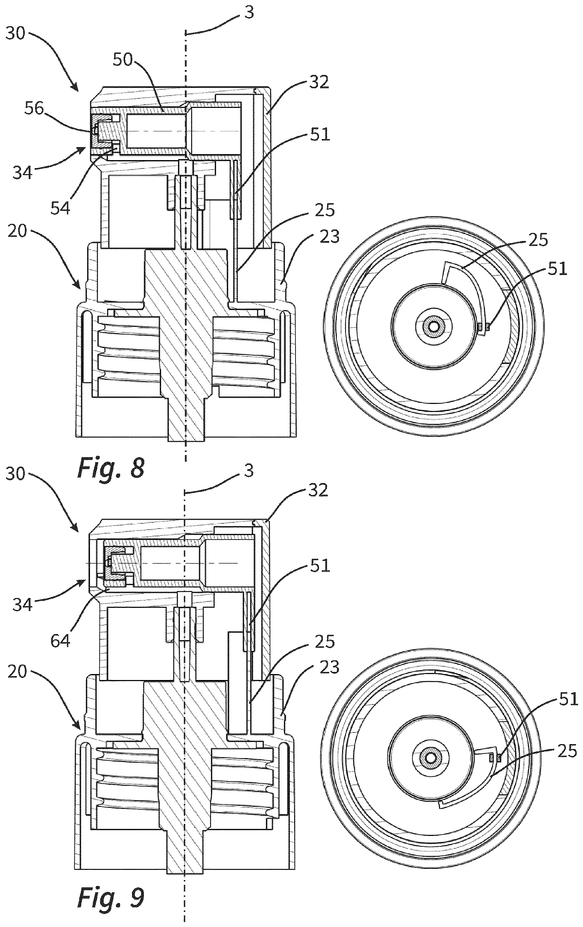

Die Fig. 8 und 9 zeigen ein drittes Ausführungsbeispiel, bei dem übereinstimmend mit der Gestaltung der Fig. 5 bis 7 das Innenteil 50 gegenüber dem Gehäuse 32 verlagerbar ist und bei dem übereinstimmend mit dem Ausführungsbeispiel der Fig. 1 bis 4 eine Wirbelkammer zur Erzeugung eines Sprühstrahls vorgesehen ist.

Allerdings handelt es sich um eine Gestaltung, bei der keine Schaltfläche vorgesehen ist. Stattdessen befindet sich an der Basiseinheit 20 ein drehfest mit dieser verbundenes Gehäuseteil 23, mit einem in den Austragkopf hineinragenden Fortsatz, der als Führungselement 25 wirkt und welcher spiralabschnittsförmig einen variablen Abstand zum Auslassstutzen 83 aufweist. Korrespondierend mit diesem Führungselement 25 ist am gegenüber dem Gehäuse 32 linear beweglichen Innenteil 50 ein Fortsatz mit einer tiefen Nut vorgesehen, der als Führungsgleiter 51 dient und in die das basisteilseitige Führungselement 25 eingreift. Wirkung dessen ist, dass durch eine Drehbewegung des Austragkopfes 30 um eine Drehachse 3 die Stellung des Innenteils 50 beeinflusst werden kann und somit die beiden beschriebenen Konfigurationen, die Sprühkonfiguration einerseits und die Tropferkonfiguration andererseits, herstellbar sind. Aufgrund der Tiefe der Nut im Führungsgleiter 51 ist dennoch gewährleistet, dass die Fortsätze das Niederdrücken des Austragkopfes nicht behindern.

## Patentansprüche

1. Flüssigkeitsspender (10), insbesondere zum Austrag pharmazeutischer oder kosmetischer Flüssigkeiten, mit den folgenden Merkmalen:
a. der Flüssigkeitsspender (10) weist eine Basiseinheit (20) mit einem Flüssigkeitsspeicher (22) zur Aufnahme von Flüssigkeit vor dem Austrag auf, und
b. der Flüssigkeitsspender (10) weist einen Austragkopf (30) zur Befestigung auf der Basiseinheit (20) des Flüssigkeitsspenders (10) auf,
c. der Flüssigkeitsspender (10) verfügt über eine Pumpeinrichtung (80), die durch eine translative Relativbewegung des Austragkopfes (30) gegenüber der Basiseinheit (20) betätigt werden kann und Flüssigkeit aus dem Flüssigkeitsspeicher (22) in den Austragkopf (30) fördert, oder der Flüssigkeitsspeicher (22) ist als Druckspeicher ausgebildet und der Flüssigkeitsspender (10) verfügt über eine Ventileinrichtung (82), das durch eine translative Relativbewegung des Austragkopfes (30) gegenüber der Basiseinheit (20) betätigt werden kann und Flüssigkeit aus dem Flüssigkeitsspeicher (22) in den Austragkopf (30) fördert, und
d. der Austragkopf (30) weist ein Gehäuse (32) auf, das zur ortsfesten oder linearbeweglichen oder linear- und drehbeweglichen Befestigung an der Basiseinheit (20) des Flüssigkeitsspenders (10) ausgebildet ist, und
e. der Austragkopf (30) weist einen Flüssigkeitsauslass (34) auf, durch den Flüssigkeit aus dem Flüssigkeitsspeicher (22) in eine umgebende Atmosphäre abgegeben werden kann, und
f. der Austragkopf (30) weist einen Flüssigkeitseinlass (38) auf, durch den hindurch Flüssigkeit aus dem Flüssigkeitsspeicher (22) zum Flüssigkeitsauslass (34) geleitet werden kann, und
g. der Austragkopf ist für den wahlweisen Austrag von Flüssigkeit einerseits in Form eines zerstäubten Sprühstrahls und andererseits in Form eines unzerstäubten Flüssigkeitsstroms oder in Form von Einzeltropfen ausgebildet,
**gekennzeichnet durch** das folgende Merkmal:
h. der Flüssigkeitsauslass (34) verfügt über einen das Gehäuse durchdringenden Auslasskanal (36),
i. innerhalb des Auslasskanals (36) ist ein Innenteil (50) angeordnet, an deren in Richtung der umgebenden Atmosphäre weisende Stirnseite (52) eine Sprühöffnung (56) am Ende eines Sprühkanals (54) vorgesehen ist,
j. das Innenteil (50) und der Auslasskanal (36) sind gegeneinander verlagerbar, so dass in einer ersten Relativstellung ein Umgehungskanal (64) zwischen einer Innenwandung des Auslasskanals (36) und einer Außenfläche des Innenteils (50) entsteht, dessen Strömungswiderstand geringer als der des Sprühkanals (54) ist, und in einer zweiten Relativstellung dieser Umgehungskanal (64) zumindest teilweise geschlossen ist, so dass der Strömungswiderstand des Umgehungskanals (64) größer als der des Sprühkanals (54) ist.

2. Flüssigkeitsspender (10) nach Anspruch 1 mit mindestens einem der folgenden Merkmalen:
a. In der ersten Relativstellung ist der Umgehungskanal (64) geschlossen, so dass Flüssigkeit nur durch den Sprühkanal (54) in die umgebende Atmosphäre gelangen kann, und/oder
b. in der zweiten Relativstellung ist ein Einlass (53) in den Sprühkanal (54) geschlossen, so dass Flüssigkeit nur durch den Umgehungskanal (64) in die umgebende Atmosphäre gelangen kann.

3. Flüssigkeitsspender (10) nach Anspruch 1 oder 2 mit einem der folgenden Merkmale:
a. die Innenwandung des Auslasskanals (36) wird unmittelbar durch Oberflächen des Gehäuses (32) gebildet und das Innenteil (50) ist gegenüber dem Gehäuse (32) verlagerbar, oder
b. die Innenwandung des Auslasskanals (36) wird durch eine gegenüber dem Gehäuse (32) verlagerbare Auslasshülse (60) gebildet und ist damit auch gegenüber dem Innenteil (50) verlagerbar, welches Teil des Gehäuses (32) ist.

4. Flüssigkeitsspender (10) nach einem der vorstehenden Ansprüche mit mindestens einem der folgenden Merkmale:
a. der Sprühkanal (54) umfasst eine Wirbelkammer (58), in die Flüssigkeit in einer vor einer Radialrichtung abweichenden Richtung einströmt,
b. das Innenteil (50) weist einen Außenwandungsabschnitt und ein darein eingesetztes Sprühbauteil (59) auf, und/oder
c. das Sprühbauteil (59) umfasst die Sprühöffnung (56) und bildet zumindest Teil einer Innenwandung der Wirbelkammer (58).

5. Flüssigkeitsspender (10) nach einem der vorstehenden Ansprüche mit dem folgenden Merkmal:
a. das Innenteil (50) und der Auslasskanal (36) sind relativ zueinander translativ beweglich, vorzugsweise linear beweglich, insbesondere vorzugsweise fluchtend mit einer Haupterstreckungsrichtung des Auslasskanals,
vorzugsweise mit dem folgenden Merkmal:
b. das Innenteil (50) oder der Auslasskanal sind über einen Gewindetrieb translativ beweglich, so dass über eine rotative Stellbewegung die Relativverlagerung erfolgt.

6. Flüssigkeitsspender (10) nach einem der vorstehenden Ansprüchen mit den folgenden Merkmalen:
a. es ist eine Schaltfläche (70) zur manuellen Betätigung vorgesehen, mittels derer die Relativlage des Auslasskanals (36) gegenüber dem Innenteil (50) veränderbar ist, und
b. die Schaltfläche (70) ist unmittelbar an einer gegenüber dem Gehäuse (32) beweglichen Auslasshülse (60) oder am gegenüber dem Gehäuse beweglichen Innenteil (50) vorgesehen.

7. Flüssigkeitsspender (10) nach einem der Ansprüche 1 bis 5 mit den folgenden Merkmalen:
a. es ist eine Schaltfläche (72) zur manuellen Betätigung vorgesehen, mittels derer die Relativlage des Auslasskanals (36) gegenüber dem Innenteil (50) veränderbar ist, und
b. die Schaltfläche (72) ist mittels eines Getriebes (74) mit dem gegenüber dem Gehäuse (32) beweglichen Auslasskanal oder mit dem gegenüber dem Gehäuse beweglichen Innenteil gekoppelt.

8. Flüssigkeitsspender (10) nach einem der Ansprüche 6 oder 7 mit dem folgenden Merkmal:
a. es ist eine Rückstellfeder (76) vorgesehen, die zwischen dem Innenteil (50) und dem Auslasskanal (36) wirkt, so dass das Innenteil (50) und der Auslasskanal (36) stets in Richtung der ersten oder der zweiten Relativstellung kraftbeaufschlagt sind und durch Kraftbeaufschlagung der Schaltfläche (72) gegen die Kraft der Rückstellfeder (76) verlagert werden.

9. Flüssigkeitsspender (10) nach einem der vorstehenden Ansprüche mit mindestens einem der folgenden Merkmale:
a. der Austragkopf (30) verfügt über ein variables Innenvolumen für Flüssigkeit, welches durch Einschieben der Auslasshülse (60) vergrößert wird, und/oder
b. der Auslasskanal (36) weist eine sich in Richtung der umgebenden Atmosphäre verjüngende Formgebung auf, und/oder
c. der Umgehungskanal (64) umgibt das Innenteil (50) ringförmig.

10. Flüssigkeitsspender (10) nach einem der vorstehenden Ansprüche, **gekennzeichnet durch** das Merkmal:
a. die Schaltfläche (70; 72) zur Relativverlagerung des Innenteils (50) und des Auslasskanals (36) gegeneinander ist auf einer der Basiseinheit (20) abgewandten Seite des Austragkopfes (30) vorgesehen,
vorzugsweise mit dem zusätzlichen Merkmal:
b. die Schaltfläche (72) ist gegenüber dem Gehäuse (32) des Austragkopfes (30) in einer Richtung beweglich, die der translativen Relativbewegungsrichtung des Austragkopfes (30) gegenüber der Basiseinheit (20) entspricht (+/- 20°).

11. Flüssigkeitsspender (10) nach einem der vorstehenden Ansprüche , **gekennzeichnet durch** das Merkmal:
a. der Austragkopf (30) ist gegenüber der Basiseinheit (20) um eine Drehachse (3) drehbar ausgebildet, und
b. es ist ein Getriebe vorgesehen, mittels die Drehbewegung des Austragkopfs (30) eine Relativverlagerung des Auslasskanals (36) gegenüber dem Innenteil (50) bewirkt,

12. Flüssigkeitsspender (10) nach Anspruch 11 mit den folgenden Merkmalen:
a. das Getriebe umfasst ein Führungselement (25) mit winkelabhängiger Beabstandung von der Drehachse (3), welches am Innenteil oder der Auslasshülse des Austragkopfes oder an der Basiseinheit (20) vorgesehen ist und
b. das Getriebe umfasst einen Führungsgleiter (51), der im Eingriff mit dem Führungselement (25) ist und der an der Basiseinheit oder am Innenteil (50) oder der Auslasshülse des Austragkopfes (30) vorgesehen ist

13. Flüssigkeitsspender (10) nach einem der vorstehenden Ansprüche mit den folgenden Merkmalen:
a. der Austragkopf (30) ist drehbar an der Basiseinheit (20) vorgesehen, und
b. der Austragkopf (30) verfügt über ein Getriebe, durch das die Relativstellung des Innenteils (50) gegenüber dem Auslasskanal (36) verstellbar ist.

## Claims

1. Liquid dispenser (10), in particular for discharging pharmaceutical or cosmetic liquids, having the following features:
a. the liquid dispenser (10) has a base unit (20) with a liquid reservoir (22) for accommodating liquid prior to discharge, and
b. the liquid dispenser (10) has a discharge head (30) for fastening to the base unit (20) of the liquid dispenser (10),
c. the liquid dispenser (10) has a pump device (80) which can be actuated by a translation movement of the discharge head (30) relative to the base unit (20) and delivers liquid from the liquid reservoir (22) into the discharge head (30), or the liquid reservoir (22) is configured as a pressure accumulator and the liquid dispenser (10) has a valve device (82) which can be actuated by a translation movement of the discharge head (30) relative to the base unit (20) and delivers liquid from the liquid reservoir (22) into the discharge head (30), and
d. the discharge head (30) has a housing (32) which is configured to be fastened to the base unit (20) in a stationary manner or linearly movable manner or linearly and rotatably movable manner, and
e. the discharge head (30) has a liquid outlet (34) through which liquid can be output from the liquid reservoir (22) into a surrounding atmosphere, and
f. the discharge head (30) has a liquid inlet (38) through which liquid can be conveyed from the liquid reservoir (22) to the liquid outlet (34), and
g. the discharge head is configured to discharge liquid on the one hand in the form of an atomized spray and on the other hand in the form of a non-atomized liquid stream or in the form of individual droplets,
**characterized by** the following features:
h. the liquid outlet (34) has an outlet channel (36) passing through the housing,
i. an inner part (50) is arranged inside the outlet channel (36), and a spray opening (56) is provided at the end of a spray channel (54) on the front face (52) of the inner part (50) facing in the direction of the surrounding atmosphere,
j. the inner part (50) and the outlet channel (36) are displaceable relative to each other such that, in a first relative position, a bypass channel (64) is produced between an inner wall of the outlet channel (36) and an outer face of the inner part (50), the flow resistance of which bypass channel (64) is lower than that of the spray channel (54), and, in a second relative position, this bypass channel (64) is at least partially closed, such that the flow resistance of the bypass channel (64) is greater than that of the spray channel (54).

2. Liquid dispenser (10) according to Claim 1, having at least one of the following features:
a. in the first relative position, the bypass channel (64) is closed, such that liquid can reach the surrounding atmosphere only through the spray channel (54), and/or
b. in the second relative position, an inlet (53) into the spray channel (54) is closed, such that liquid can reach the surrounding atmosphere only through the bypass channel (64).

3. Liquid dispenser (10) according to Claim 1 or 2, having one of the following features:
a. the inner wall of the outlet channel (36) is formed directly by surfaces of the housing (32), and the inner part (50) is displaceable relative to the housing (32), or
b. the inner wall of the outlet channel (36) is formed by an outlet sleeve (60) displaceable relative to the housing (32) and is thus also displaceable relative to the inner part (50), which is part of the housing (32).

4. Liquid dispenser (10) according to one of the preceding claims, having at least one of the following features:
a. the spray channel (54) comprises a vortex chamber (58) into which liquid flows in a direction deviating from a radial direction,
b. the inner part (50) has an outer wall portion and a spray component (59) installed therein, and/or
c. the spray component (59) comprises the spray opening (56) and forms at least part of an inner wall of the vortex chamber (58).

5. Liquid dispenser (10) according to one of the preceding claims, having the following feature:
a. the inner part (50) and the outlet channel (36) are movable in translation relative to each other, preferably linearly movable, particularly preferably in alignment with a main direction of extent of the outlet channel,
preferably having the following feature:
b. the inner part (50) and the outlet channel are movable in translation via a threaded drive, such that the relative displacement is effected via a rotary adjustment movement.

6. Liquid dispenser (10) according to one of the preceding claims, having the following features:
a. a button (70) for manual actuation is provided, by means of which the position of the outlet channel (36) relative to the inner part (50) can be changed, and
b. the button (70) is provided directly on an outlet sleeve (60) movable relative to the housing (32) or on the inner part (50) movable relative to the housing.

7. Liquid dispenser (10) according to one of Claims 1 to 5, having the following features:
a. a button (72) for manual actuation is provided, by means of which the position of the outlet channel (36) relative to the inner part (50) can be changed, and
b. the button (72) is coupled by means of a transmission (74) to the outlet channel movable relative to the housing (32), or to the inner part movable relative to the housing.

8. Liquid dispenser (10) according to either of Claims 6 and 7, having the following feature:
a. a restoring spring (76) is provided which acts between the inner part (50) and the outlet channel (36) such that the inner part (50) and the outlet channel (36) are subjected to force always in the direction of the first or second relative position and, when force is applied at the push button (72), are displaced counter to the force of the restoring spring (76).

9. Liquid dispenser (10) according to one of the preceding claims, having at least one of the following features:
a. the discharge head (30) has a variable inner volume for liquid, which volume is increased by insertion of the outlet sleeve (60), and/or
b. the outlet channel (36) has a shape that narrows in the direction of the surrounding atmosphere, and/or
c. the bypass channel (64) surrounds the inner part (50) in a ring shape.

10. Liquid dispenser (10) according to one of the preceding claims, **characterized by** the feature:
a. the button (70; 72) for the displacement of the inner part (50) and of the outlet channel (36) relative to each other is provided on a side of the discharge head (30) facing away from the base unit (20),
preferably having the additional feature:
b. the button (72) is movable relative to the housing (32) of the discharge head (30) in a direction that corresponds (+/-20°) to the direction of the translation movement of the discharge head (30) relative to the base unit (20) .

11. Liquid dispenser (10) according to one of the preceding claims, **characterized by** the features:
a. the discharge head (30) is rotatable relative to the base unit (20) about a rotation axis (3), and
b. a transmission is provided by means of which the rotation movement of the discharge head (30) causes a displacement of the outlet channel (36) relative to the inner part (50).

12. Liquid dispenser (10) according to Claim 11, having the following features:
a. the transmission comprises a guide element (25) with angle-dependent spacing from the rotation axis (3), which guide element (25) is provided on the inner part or the outlet sleeve of the discharge head or on the base unit (20), and
b. the transmission comprises a guide slide (51) which is in engagement with the guide element (25) and which is provided on the base unit or on the inner part (50) or the outlet sleeve of the discharge head (30).

13. Liquid dispenser (10) according to one of the preceding claims, having the following features:
a. the discharge head (30) is provided rotatably on the base unit (20), and
b. the discharge head (30) has a transmission by which the position of the inner part (50) relative to the outlet channel (36) is adjustable.

## Revendications

1. Distributeur de liquide (10), en particulier pour distribuer des liquides pharmaceutiques ou cosmétiques, comprenant les caractéristiques suivantes :
a. le distributeur de liquide (10) présente une unité de base (20) avec un réservoir de liquide (22) pour recevoir le liquide avant sa distribution, et
b. le distributeur de liquide (10) présente une tête de décharge (30) devant être fixée sur l'unité de base (20) du distributeur de liquide (10),
c. le distributeur de liquide (10) dispose d'un dispositif de pompe (80) qui peut être actionné par un mouvement de translation relatif de la tête de distribution (30) par rapport à l'unité de base (20) et qui refoule du liquide hors du réservoir de liquide (22) dans la tête de décharge (30), ou le réservoir de liquide (22) est réalisé sous forme de réservoir sous pression, et le distributeur de liquide (10) dispose d'un dispositif de soupape (82), qui peut être actionné par un mouvement de translation relatif de la tête de décharge (30) par rapport à l'unité de base (20) et refoule du liquide hors du réservoir de liquide (22) dans la tête de décharge (30), et
d. la tête de décharge (30) présente un boîtier (32) qui est réalisé pour être fixé de manière fixe ou déplaçable linéairement ou de manière déplaçable linéairement et en rotation à l'unité de base (20) du distributeur de liquide (10), et
e. la tête de décharge (30) présente une sortie de liquide (34) à travers laquelle du liquide peut être délivré hors du réservoir de liquide (22) dans une atmosphère environnante, et
f. la tête de décharge (30) présente une entrée de liquide (38) à travers laquelle du liquide peut être guidé hors du réservoir de liquide (22) jusqu'à la sortie de liquide (34), et
g. la tête de décharge est réalisée pour la décharge sélective de liquide d'une part sous forme d'un jet de pulvérisation pulvérisé et d'autre part sous forme d'un courant de liquide non pulvérisé ou sous forme de gouttes individuelles,
**caractérisé par** les caractéristiques suivantes :
h. la sortie de liquide (34) dispose d'un canal de sortie (36) traversant le boîtier,
i. à l'intérieur du canal de sortie (36) est disposée une partie interne (50) au niveau du côté frontal (52) de laquelle, tourné dans la direction de l'atmosphère environnante, est prévue une ouverture de pulvérisation (56) à l'extrémité d'un canal de pulvérisation (54),
j. la partie interne (50) et le canal de sortie (36) peuvent être déplacés l'un par rapport à l'autre, de telle sorte que dans une première position relative, un canal de contournement (64) soit formé entre une paroi interne du canal de sortie (36) et une surface extérieure de la partie interne (50), dont la résistance à l'écoulement est inférieure à celle du canal de pulvérisation (54), et dans une deuxième position relative, ce canal de contournement (64) soit au moins en partie fermé, de telle sorte que la résistance à l'écoulement du canal de contournement (64) soit supérieure à celle du canal de pulvérisation (54).

2. Distributeur de liquide (10) selon la revendication 1, comprenant au moins l'une des caractéristiques suivantes :
a. dans la première position relative, le canal de contournement (64) est fermé, de telle sorte que du liquide puisse seulement parvenir à travers le canal de pulvérisation (54) dans l'atmosphère environnante, et/ou
b. dans la deuxième position relative, une entrée (53) dans le canal de pulvérisation (54) est fermée, de telle sorte que le liquide puisse seulement parvenir à travers le canal de contournement (64) dans l'atmosphère environnante.

3. Distributeur de liquide (10) selon la revendication 1 ou 2, comprenant l'une des caractéristiques suivantes :
a. la paroi interne du canal de sortie (36) est formée directement par des surfaces du boîtier (32) et la partie interne (50) est déplaçable par rapport au boîtier (32), ou
b. la paroi interne du canal de sortie (36) est formée par une douille de sortie (60) déplaçable par rapport au boîtier (32), et par conséquent est également déplaçable par rapport à la partie interne (50), qui fait partie du boîtier (32).

4. Distributeur de liquide (10) selon l'une quelconque des revendications précédentes, comprenant au moins l'une des caractéristiques suivantes :
a. le canal de pulvérisation (54) comprend une chambre de tourbillonnement (58) dans laquelle du liquide afflue dans une direction s'écartant d'une direction radiale,
b. la partie interne (50) présente une portion de paroi extérieure et un composant de pulvérisation (59) inséré dans celle-ci, et/ou
c. le composant de pulvérisation (59) comprend l'ouverture de pulvérisation (56) et forme au moins une partie d'une paroi interne de la chambre de tourbillonnement (58).

5. Distributeur de liquide (10) selon l'une quelconque des revendications précédentes, comprenant la caractéristique suivante :
a. la partie interne (50) et le canal de sortie (36) peuvent être déplacés en translation l'un par rapport à l'autre, de préférence de manière linéaire, en particulier de préférence en alignement avec une direction d'étendue principale du canal de sortie,
de préférence comprenant la caractéristique suivante :
b. la partie interne (50) ou le canal de sortie peuvent être déplacés en translation par le biais d'une vis d'entraînement, de telle sorte que le déplacement relatif s'effectue par le biais d'un mouvement de réglage rotatif.

6. Distributeur de liquide (10) selon l'une quelconque des revendications précédentes, comprenant les caractéristiques suivantes :
a. il est prévu une surface de commutation (70) pour l'actionnement manuel, au moyen de laquelle la position relative du canal de sortie (36) par rapport à la partie interne (50) peut être modifiée, et
b. la surface de commutation (70) est prévue directement au niveau d'une douille de sortie (60) déplaçable par rapport au boîtier (32) ou est prévue au niveau de la partie interne (50) déplaçable par rapport au boîtier.

7. Distributeur de liquide (10) selon l'une quelconque des revendications 1 à 5, comprenant les caractéristiques suivantes :
a. il est prévu une surface de commutation (72) pour l'actionnement manuel, au moyen de laquelle la position relative du canal de sortie (36) par rapport à la partie interne (50) peut être modifiée, et
b. la surface de commutation (72) est accouplée au moyen d'un mécanisme (74) au canal de sortie déplaçable par rapport au boîtier (32) ou est accouplée à la partie interne déplaçable par rapport au boîtier.

8. Distributeur de liquide (10) selon l'une quelconque des revendications 6 ou 7, comprenant la caractéristique suivante :
a. il est prévu un ressort de rappel (76), qui agit entre la partie interne (50) et le canal de sortie (36), de telle sorte que la partie interne (50) et le canal de sortie (36) soient toujours sollicités par une force dans la direction de la première ou de la deuxième position relative, et soient déplacés par une sollicitation par une force de la surface de commutation (72) à l'encontre de la force du ressort de rappel (76).

9. Distributeur de liquide (10) selon l'une quelconque des revendications précédentes, comprenant au moins l'une des caractéristiques suivantes :
a. la tête de décharge (30) dispose d'un volume interne variable pour le liquide, qui est accru en insérant la douille de sortie (60), et/ou
b. le canal de sortie (36) présente une forme se rétrécissant dans la direction de l'atmosphère environnante, et/ou
c. le canal de contournement (64) entoure la partie interne (50) sous forme annulaire.

10. Distributeur de liquide (10) selon l'une quelconque des revendications précédentes, **caractérisé par** la caractéristique:
a. la surface de commutation (70 ; 72) pour le déplacement relatif de la partie interne (50) et du canal de sortie (36) l'un par rapport à l'autre est prévue sur un côté de la tête de décharge (30) opposé à l'unité de base (20),
de préférence comprenant la caractéristique supplémentaire :
b. la surface de commutation (72) est déplaçable par rapport au boîtier (32) de la tête de décharge (30) dans une direction qui correspond à la direction de déplacement en translation relatif de la tête de décharge (30) par rapport à l'unité de base (20) (± 20°).

11. Distributeur de liquide (10) selon l'une quelconque des revendications précédentes, **caractérisé par** les caractéristiques suivantes :
a. la tête de décharge (30) est réalisée de manière à pouvoir tourner autour d'un axe de rotation (3) par rapport à l'unité de base (20), et
b. il est prévu un mécanisme au moyen duquel le mouvement de rotation de la tête de décharge (30) provoque un déplacement relatif du canal de sortie (36) par rapport à la partie interne (50).

12. Distributeur de liquide (10) selon la revendication 11, comprenant les caractéristiques suivantes :
a. le mécanisme comprend un élément de guidage (25) avec un espacement de l'axe de rotation (3) dépendant de l'angle, qui est prévu au niveau de la partie interne ou de la douille de sortie de la tête de décharge ou au niveau de l'unité de base (20) et
b. le mécanisme comprend un coulisseau de guidage (51) qui est en prise avec l'élément de guidage (25) et qui est prévu au niveau de l'unité de base ou au niveau de la partie interne (50) ou de la douille de sortie de la tête de décharge (30) .

13. Distributeur de liquide (10) selon l'une quelconque des revendications précédentes, comprenant les caractéristiques suivantes :
a. la tête de décharge (30) est prévue de manière à pouvoir tourner sur l'unité de base (20), et
b. la tête de décharge (30) dispose d'un mécanisme par le biais duquel la position relative de la partie interne (50) par rapport au canal de sortie (36) peut être réglée.
